# EUROPEAN PATENT APPLICATION

(11) **EP 2 163 209 A1**
(43) Date of publication of application: **17.03.2010**
(21) Application number: 08105350.6
(22) Date of filing: 15.09.2008
(51) Int. Cl.: A61B 17/072, A61B 17/068

(54) **Lockout mechanism for a surgical stapler**

(71) Applicant: Weng, Zhiqiang, Pingjiang District Jiangsu Suzhou (CN)
(72) Inventor: Weng, Zhiqiang, Pingjiang District Jiangsu Suzhou (CN)
(74) Representative: Molnia, David

(57) **Abstract**

The invention relates to a surgical stapler for applying at least two rows of staples, said surgical stapler comprising a cartridge housing said rows of staples and having a slot extending along the cartridge between two rows of staples, said surgical stapler further comprising a cutting means movable along said slot and a lockout mechanism for preventing movement of said cutting means, wherein said lockout mechanism comprises a blocking member movable between a first position, in which the blocking member blocks movement of said cutting means, and a second position, in which the blocking member permits movement of said cutting means, and a biasing member for biasing said blocking member towards said first position and wherein said cartridge comprises a release member for moving said blocking member towards said second position upon insertion of said cartridge into said surgical stapler.

## Description

The invention relates to a surgical stapler for applying a plurality of surgical staples in rows and for cutting the stapled tissue between two of the staple rows. In particular, the invention relates to an improved lockout feature for a surgical stapler preventing the stapler to be used without staples.

Surgical staplers are well known medical tools especially for closure of wounds but also for closure of internal organs prior to transection or resection. Surgical staplers often are used with removable and disposable cartridges housing the staples in essentially parallel rows and having a slot between two of these rows for guiding a knife. The surgeon can thus perform closure and transection of the organ in a single step.

It is therefore of utmost importance that the knife of the stapler can only be activated and moved to cut the organ after the organ has already been closed by means of one or more rows of staples on both sides of the knife. Otherwise the patient could be severely harmed by leakage of fluids from the resected organ and his life put in danger.

There exist a number of lockout mechanisms which prevent movement of the knife if no or a previously used cartridge is present in the stapler.

From EP 0 793 943 B1 a surgical stapler is known where the knife is biased upwards by a leaf spring as long as no cartridge is inserted. Blocking features on the knife and on a knife guiding block then prevent the longitudinal movement of the knife. A rotatable member on the cartridge engages the knife and moves it downwards, thus releasing the blocking features and allowing longitudinal movement of the knife. During its longitudinal movement, the knife rotates the rotatable member to an inactive position where it no longer can engage the knife.

From US 5,332,142 a surgical stapler is known where the knife is lifted to an upper position clear of an obstructing element in the stapler by a lockout feature of the cartridge. Longitudinal movement of the knife deactivates the lockout feature and the knife, now in a lower position, is subsequently prevented from being moved forward a second time.

Allowing the knife to move in a direction other than the longitudinal direction of the stapler, however, complicates the knife holding mechanism and prevents a secure and narrow guiding of the knife in the stapler.

It is thus the object of the present invention to provide a surgical stapler with a lockout mechanism preventing usage of the stapler with no or a used cartridge present and simultaneously having a secure guiding of the knife.

This object is solved by a surgical stapler having a lockout mechanism according to the present invention.

A surgical stapler according to the present invention can be used in combination with a staple cartridge housing the staples to be inserted into tissue in at least two substantially parallel rows and having a slot extending along the staple cartridge between two rows of staples. Staples can be arranged in the staple cartridge in more than two rows, wherein an even number of rows is preferred such that it is possible to have the same number of rows on both sides of the slot.

The surgical stapler comprises a cutting means that is movable along said slot to cut stapled tissue. Preferably, the cutting means is initially located in a position, in which the distal end of the cutting means is located proximally of the rows of staples in the staple cartridge, to ensure that tissue is not cut before it has been stapled. The surgical stapler further comprises a lockout mechanism for preventing movement of the cutting means into and along said slot while no or a used staple cartridge is inserted into the surgical stapler.

The lockout mechanism of the surgical stapler comprises a blocking member and a biasing member. The blocking member is movable between a first position, in which the blocking member blocks longitudinal movement of said cutting means, and a second position, in which the blocking member permits longitudinal movement of said cutting means. Said biasing member biases said blocking member towards said first position such that longitudinal movement of said cutting means is prevented until said blocking member is actively moved into the second position by overcoming the biasing force of the biasing member.

The staple cartridge comprises a release member that engages said blocking member of said lockout mechanism and moves said blocking member from said first position into said second position when the staple cartridge is being inserted into the surgical stapler and holds said blocking member in said second position as long as said cutting means of said surgical stapler is not moved into said slot of said staple cartridge.

In a preferred embodiment of the present invention, the blocking member is rotationally mounted in the surgical stapler.

In a further preferred embodiment of the present invention, the blocking member comprises a blocking portion and the cutting member comprises a shoulder. Said blocking portion and said shoulder are arranged such that the blocking portion prevents distal movement of the cutting means by contacting said shoulder of the cutting means when said blocking member is in the first position.

In a further preferred embodiment of the present invention, the surgical stapler comprises a guiding element for guiding said cutting means. The guiding element provides a guiding channel having dimensions adapted to the dimensions of said cutting means for ensuring a secure guiding of said cutting means. The guiding element prevents lateral movement (i.e. movement in a direction essentially perpendicular to the longitudinal direction of the surgical stapler) of said cutting means as much as possible without interfering with the longitudinal movement of said cutting means.

In a further preferred embodiment, said blocking member is movable in a direction essentially perpendicular to the longitudinal direction of the stapler and said guiding element also guides said blocking member during its movement from the first position into the second position and vice versa. For guiding said blocking member, said guiding element provides a passage extending essentially perpendicular to said passage for guiding said cutting means.

In a further preferred embodiment, said blocking member is an essentially planar element. The plane defined by the essentially planar element is arranged essentially perpendicular to the longitudinal direction of the stapler. In an especially preferred embodiment said biasing member is a spring engaging a peripheral end of the blocking member.

In a further preferred embodiment, said blocking member and/or said biasing member are wire sections. In an especially preferred embodiment, said blocking member and said biasing member are wire portions of an integral wire constituting an essentially U-shaped form, wherein said biasing member is constituted by the legs of said U and said blocking member is constituted by the wire portion connecting said legs of said U. The free ends of said U can be attached to said guiding element or they can be connected to end portions of the integral wire which are then attached to said guiding element or to another part of the surgical stapler.

The present invention further relates to a staple cartridge for use with a surgical stapler as defined above. As explained above, a staple cartridge according to the present invention comprises a release member that engages said blocking member of said lockout mechanism and moves said blocking member from said first position into said second position when the staple cartridge is being inserted into the surgical stapler and holds said blocking member in said second position as long as said cutting means of said surgical stapler is not moved into said slot of said staple cartridge.

In a preferred embodiment, said release member of said staple cartridge is integrally formed with the body of said staple cartridge, e.g. by means of injection moulding.

In a further preferred embodiment, said release member of said staple cartridge is a separate component which is assembled onto said staple cartridge when said staple cartridge is being inserted into the surgical stapler. In the assembled state, said release member is preferably arranged between said staple cartridge and the surgical stapler so that said release member is fixed in position and cannot work loose.

In a still further preferred embodiment, said release member of said staple cartridge is deactivatable by means of longitudinal movement of said cutting means of said surgical stapler.

In a further preferred embodiment of the staple cartridge, longitudinal movement of said cutting means in the distal direction causes the distal end of said cutting means to contact a portion of said release member. Upon further movement of said cutting means in the distal direction, said release member is plastically bent with respect to said body of said staple cartridge and thus said blocking member is released. When said cutting means returns to its initial position, said biasing member biases said blocking member towards said first position and said blocking member prevents any further attempts to move said cutting means in longitudinal direction. By plastically bending it, said release member is permanently deformed, and thus permanently deactivated so that subsequently inserting a used staple cartridge, which comprises a permanently deformed release member, into the surgical stapler will not result in said blocking member moving to said second position.

For a more complete understanding of the present invention and its features and advantages, reference is made to the following description, taken in conjunction with the accompanying drawings, in which:
Fig. 1 illustrates a perspective view of a lockout mechanism of a surgical stapler according to a first embodiment of the present invention,
Fig. 2 illustrates a perspective view of the lockout mechanism according to Fig. 1 which is engaged by a release member of a staple cartridge according to the first embodiment of the present invention of Fig. 1 in a state ready for operation,
Fig. 3 illustrates a perspective view of the components according to Fig. 2 in a state during operation,
Fig. 4 illustrates a perspective view of the components according to Fig. 2 in a state after completing operation,
Fig. 5 illustrates a section view through the components according to Fig. 2 including the staple cartridge,
Fig. 6 illustrates a perspective view of a lockout mechanism of a surgical stapler according to a second embodiment of the present invention wherein the lockout mechanism is formed by a wire,
Fig. 7 illustrates a section view through the lockout mechanism according to Fig. 6 being engaged by a release member of a staple cartridge according to the second embodiment of the present invention in a state ready for operation,
Fig. 8 illustrates a section view through the components according to Fig. 7 in a state during operation,
Fig. 9 illustrates a section view through the components according to Fig. 7 in a state after completing operation,
Fig. 10 illustrates a perspective view of the cutting means and the release member according to a third embodiment of the present invention wherein the release member is formed as a separate component,
Fig. 11 illustrates a perspective view of the components according to Fig. 10 including the lockout mechanism in a state ready for operation,
Fig. 12 illustrates a perspective view of the components according to Fig. 11 in a state during operation,
Fig. 13 illustrates a perspective view of the components according to Fig. 11 in a state after completing operation,
Fig. 14 illustrates a perspective view of the release member according to Fig. 10 which is assembled onto a staple cartridge according to the third embodiment of the present invention of Fig. 10,
Fig. 15 illustrates a perspective view of the components according to Fig. 14 which are inserted into a surgical stapler according to the third embodiment of the present invention of Fig. 10,
Fig. 16 illustrates a side view of the locking mechanism according to a fourth embodiment of the present invention in a state ready for operation,
Fig. 17 illustrates a side view of the locking mechanism according to the fourth embodiment of Fig. 16 in a state after completing operation,
Fig. 18 illustrates a perspective view of the blocking member of the fourth embodiment of Fig. 16, and
Fig. 19 illustrates a perspective view of the release member of the fourth embodiment of Fig. 16.

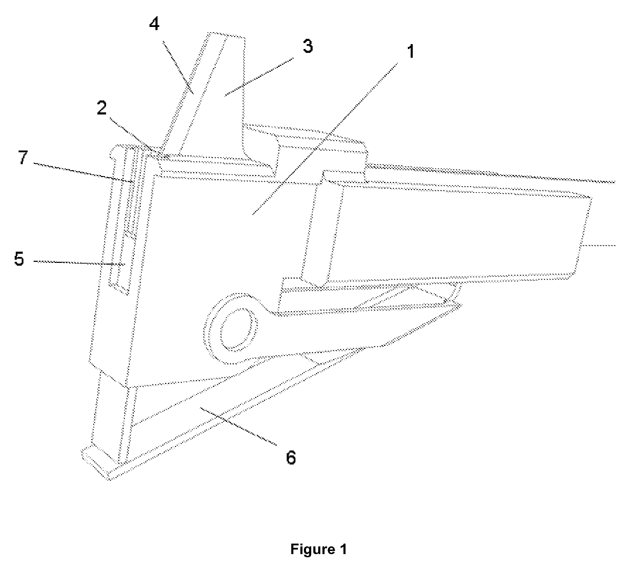

In Fig. 1 a perspective view of a lockout mechanism of a surgical stapler according to a first embodiment of the present invention is depicted. For purpose of clarity, only the guiding element 1, the cutting means 3, and the lockout mechanism of the surgical stapler are shown. The guiding element 1 provides a guiding channel 2 having dimensions adapted to the dimensions of the cutting means 3 for ensuring a secure guiding of the cutting means 3. The cutting means 3 comprises a blade 4 at its distal end. The lockout mechanism comprises a blocking member 5 and a biasing member 6. In this embodiment of the present invention, the blocking member 5 is an essentially planar element. For guiding the blocking member 5, the guiding element 1 provides a passage 7 extending essentially perpendicular to the guiding channel 2. Within the passage 7, the blocking member 5 is movable from a first position into a second position and vice versa. In Fig. 1, the blocking member 5 is shown in the second position. In the second position, the blocking member 5 permits longitudinal movement of the cutting means 3 whereas in the first position the blocking member 5 blocks longitudinal movement of the cutting means 3. The biasing member 6 is a leaf spring engaging a peripheral end of the blocking member 5. The biasing member 6 biases the blocking member 5 towards the first position until the blocking member 5 is actively moved into the second position by overcoming the biasing force of the biasing member 6.

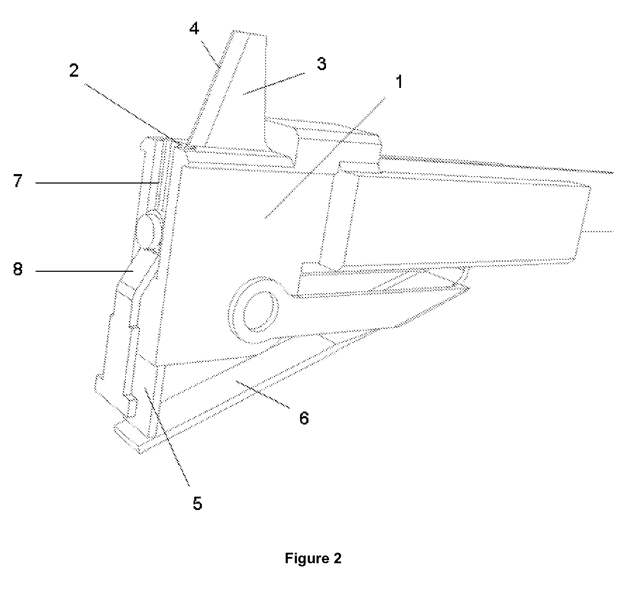

Fig. 2 is almost a copy of Fig. 1, except that a release member 8 is shown. The release member 8 engages the blocking member 5 and holds the blocking member 5 in the second position against the biasing force of the biasing member 6. The release member 8 is itself part of a staple cartridge according to the first embodiment of the present invention of Fig. 1. The staple cartridge again is not shown for purposes of clarity. In the depicted state, the surgical stapler is ready for operation.

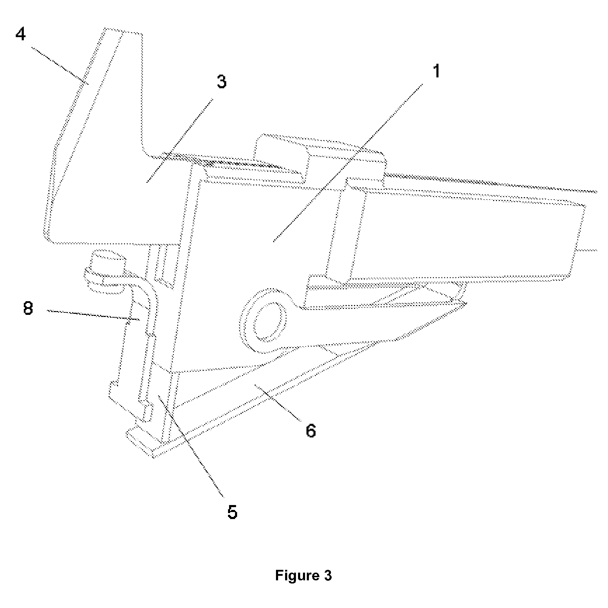

Fig. 3 illustrates a perspective view of the components according to Fig. 2 in a state during operation of the surgical stapler. The cutting means 3 is advanced in distal direction, thus deflecting the release member 8 which allows the release member 8 to release the blocking member 5. The blocking member 5, however, is unable to slide upwards under spring bias into the first position as it is retained in the second position by the presence of the advancing cutting means 3.

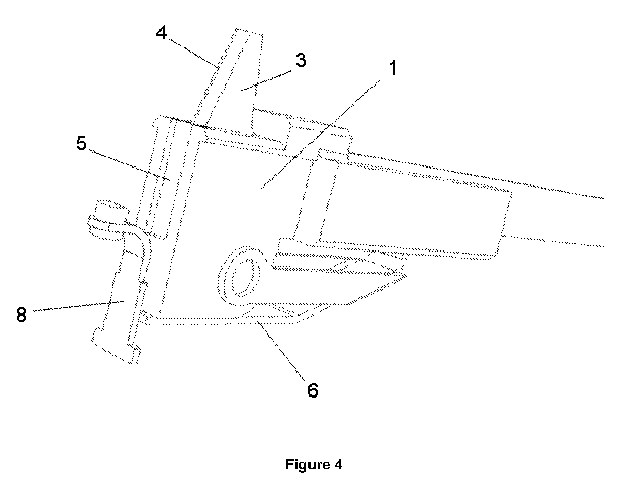

Fig. 4 illustrates a perspective view of the components according to Fig. 2 when the stapling process is complete. The cutting means 3 has returned to the retracted position. The blocking member 5 is now able to slide upwards into the first position, under the influence of the biasing member 6, thus preventing any repeated deployment of the cutting means 3. Since the release member 8 is plastically deformed, the cutting means 3 can only be deployed when a new staple cartridge is inserted having a release member 8 which has not been plastically deformed or bent.

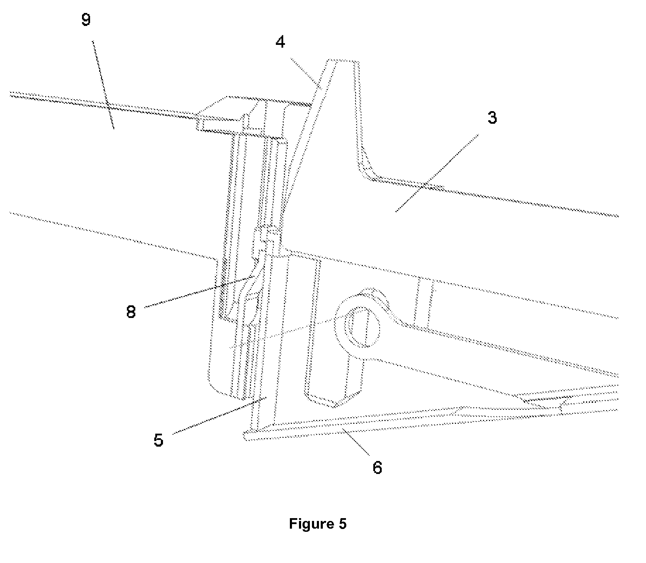

Fig. 5 illustrates a section view through the components according to Fig. 2 including the staple cartridge 9. It can be seen that the lower end of the release member 8 is embedded in the staple cartridge 9 while its upper portion engages the blocking member 5, holding the blocking member 5 in the second position.

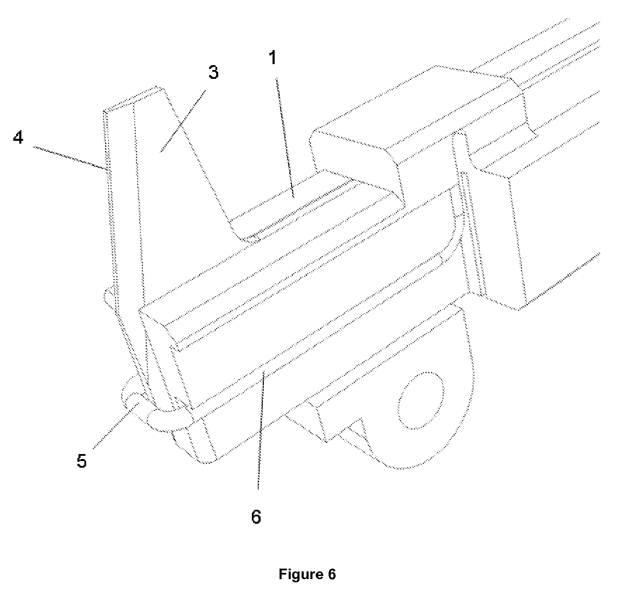

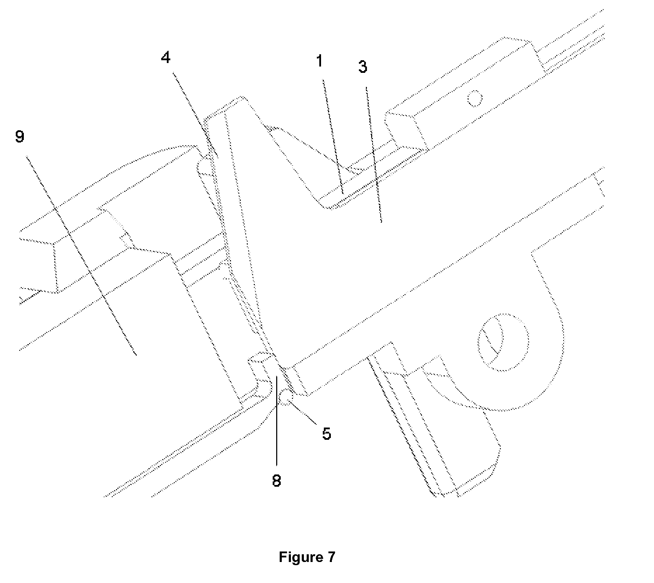

Fig. 6 illustrates a perspective view of a lockout mechanism of a surgical stapler according to a second embodiment of the present invention. This embodiment is similar to that described above except that the blocking member 5 and the biasing member 6 are replaced by a single piece of wire 5, 6 with spring-like properties. The wire 5, 6 is shown mounted in the guiding element 1 and obstructing advancement of the cutting means 3. Other than these differences, this embodiment operates in a very similar manner, as is depicted in Figs. 7-9.

Fig. 7 illustrates a section view through the lockout mechanism according to Fig. 6 being engaged by a release member 8 of a staple cartridge 9 according to the second embodiment of the present invention. The wire 5 is shown captured by the release member 8 and retained in the second position, in which the wire 5 permits longitudinal movement of the cutting means 3. The release member 8 is moulded integrally with the body of the staple cartridge 9. In the depicted state, the surgical stapler is ready for operation.

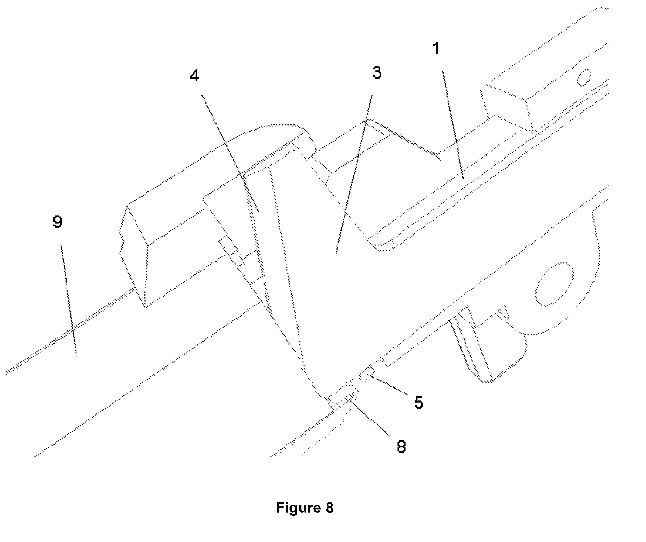

Fig. 8 illustrates a section view through the components according to Fig. 7 in a state during operation of the surgical stapler. The cutting means 3 is advanced in distal direction, thus bending the release member 8 towards the left of Fig. 8. This action disengages the wire 5 from the release member 8. The wire 5 is still held down in the second position by the lower surface of the cutting means 3.

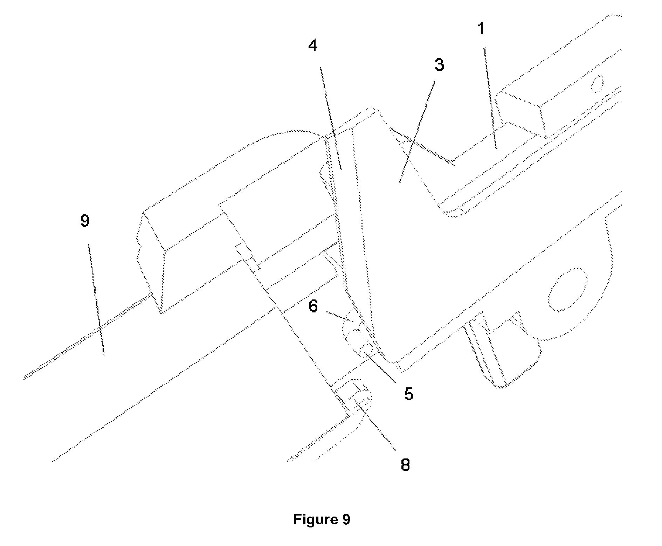

Fig. 9 illustrates a section view through the components according to Fig. 7 in a state after completing operation. The cutting means 3 is shown after being completely withdrawn. The release member 8 remains deformed and the wire 5, 6 springs into the first position, thus preventing the cutting means 3 from being advanced again. Inserting a new staple cartridge 9 into the surgical stapler will again capture the wire 5, 6 in the release member 8 of the staple cartridge 9 in readiness for a new stapling action.

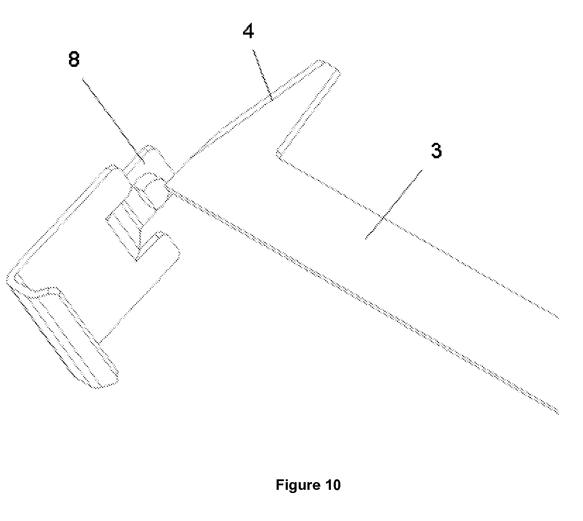

Fig. 10 illustrates a perspective view of the cutting means 3 and the release member 8 according to a third embodiment of the present invention wherein the release member 8 is formed as a separate component. By forming the release member 8 as a separate component, the injection moulding of the staple cartridge does not need to be modified, which would incur large costs. The figure depicts the cutting means 3 positioned relative to the release member 8 prior to, and on completion of, operation of the surgical stapler. The other components are omitted for purpose of clarity. The release member 8 is preferably made from a steel stamping. For engaging the blocking member, not shown in this figure, the release member 8 comprises a button riveted into sheet. By conditioning the sheet to a malleable temper, the release member 8 may bend plastically without breaking.

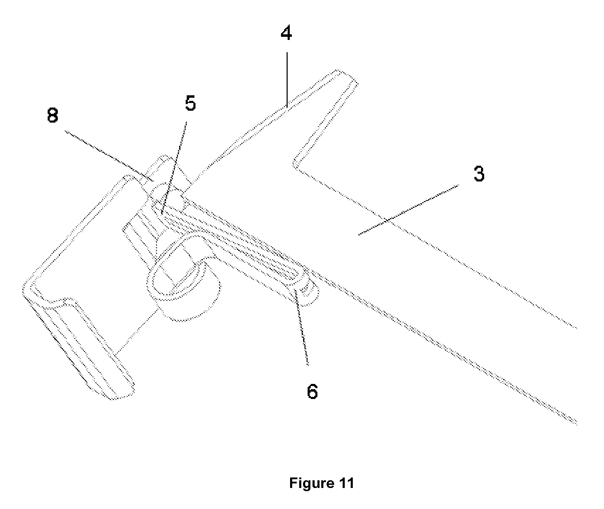

Fig. 11 illustrates a perspective view of the components according to Fig. 10 including the lockout mechanism. In this third embodiment, the blocking member 5 and the biasing member 6 of the lockout mechanism are formed by a single piece made from a spring steel stamping. The lockout mechanism 5, 6 is mounted in the guiding element 1, not shown. The release member 8 engages the blocking member 5 and pulls the blocking member 5 against the biasing force of the biasing member 6 into the second position, in which the blocking member 5 permits longitudinal movement of the cutting means 3. In the depicted state, the surgical stapler is ready for operation.

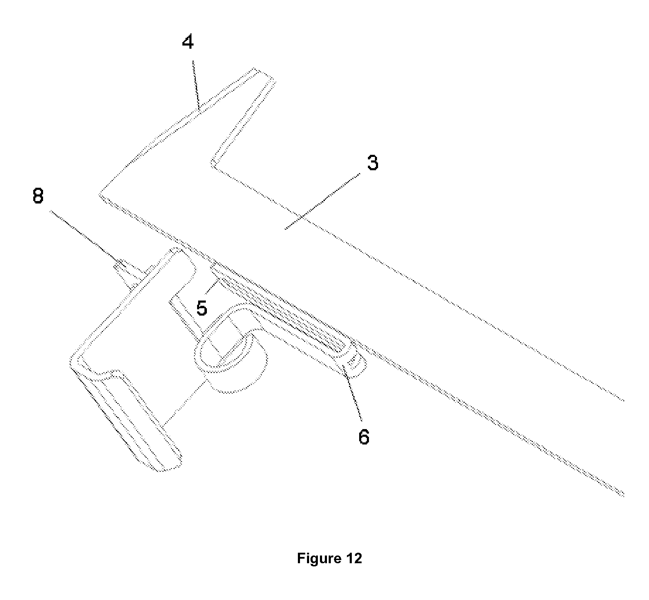

Fig. 12 illustrates a perspective view of the components according to Fig. 11 in a state during operation of the surgical stapler. The cutting means 3 is advanced in distal direction, thus deflecting the release member 8 which allows the release member 8 to release the blocking member 5. The blocking member 5, however, is unable to slide upwards under spring bias into the first position as it is retained in the second position by the presence of the advancing cutting means 3.

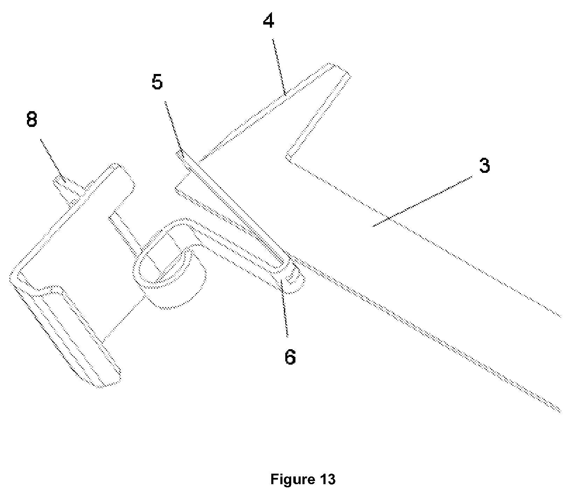

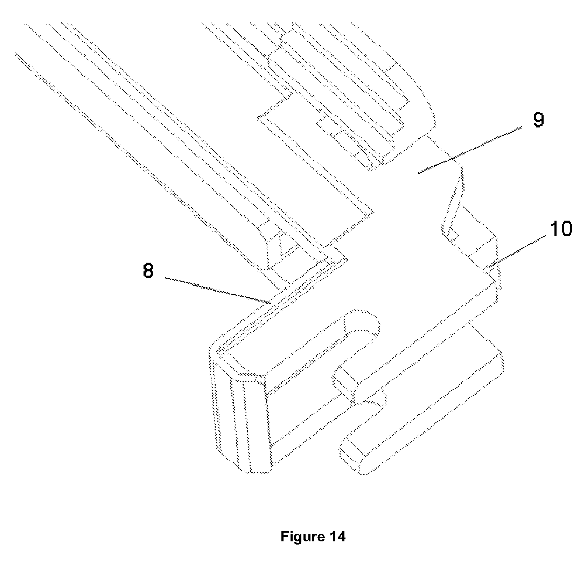

Fig. 13 illustrates a perspective view of the components according to Fig. 11 when the stapling process is complete. The cutting means 3 has returned to the retracted position. The blocking member 5 is now able to slide upwards into the first position, under the influence of the biasing member 6, thus preventing any repeated deployment of the cutting means 3. Since the release member 8 is plastically deformed, the cutting means 3 can only be deployed again when the release member 8 is replaced by a new release member 8. For this, the empty staple cartridge, not shown, has to be removed, as is evident from Figs. 14-15.

Fig. 14 illustrates a perspective view of the release member 8 according to Fig. 10 which is assembled onto a staple cartridge 9. The release member 8 crimps around the legs of the staple cartridge 9. In this figure, a slot 10 extending along the staple cartridge 9 between the rows of staples is shown. The cutting means, not shown, is movable along the slot 10 to cut stapled tissue.

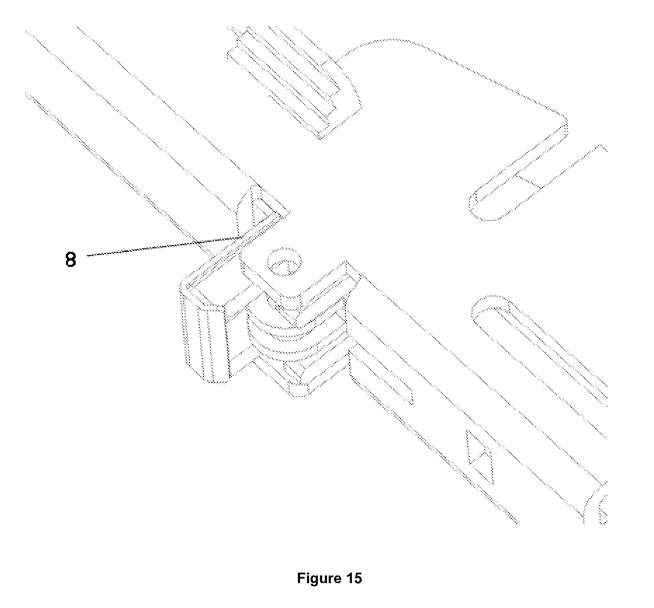

Fig. 15 illustrates a perspective view of the components according to Fig. 14 which are inserted into the surgical stapler. As can be seen from this figure, the release member 8 is arranged between the staple cartridge and the surgical stapler so that the release member 8 is fixed in position and cannot work itself loose.

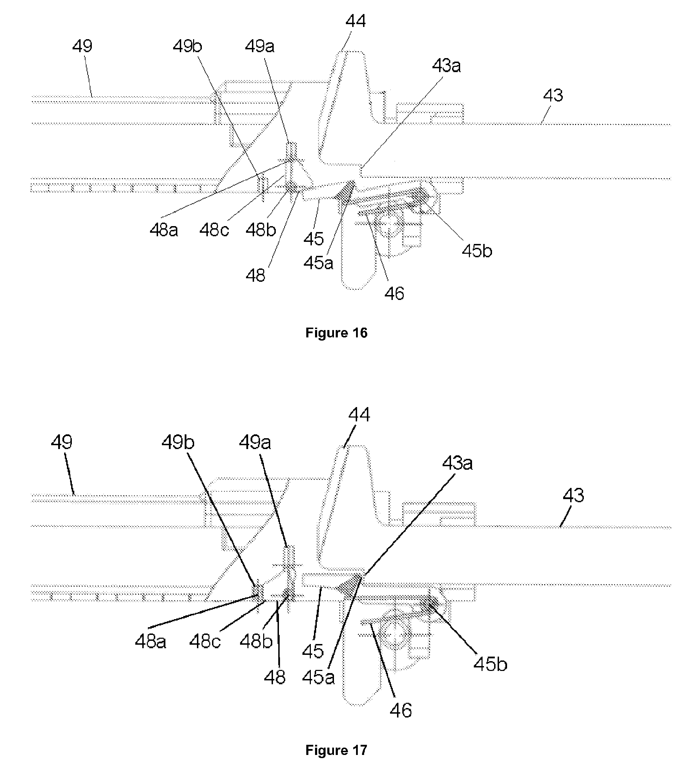

A fourth embodiment of the present invention is illustrated in Figs. 16 - 19. Figs. 16 and 17 show a side view of the lockout mechanism of the fourth embodiment in a state ready for operation and after completing operation, respectively. In these drawings, only components that are relevant for operation of the lockout mechanism are shown. Other components (like housing or guiding elements) have been omitted for the sake of clarity.

In Fig. 16, the cutting means 43 comprising a blade 44 at its distal end is shown in its initial position before operation of the surgical stapler. A cartridge 49 has been inserted into the surgical stapler.

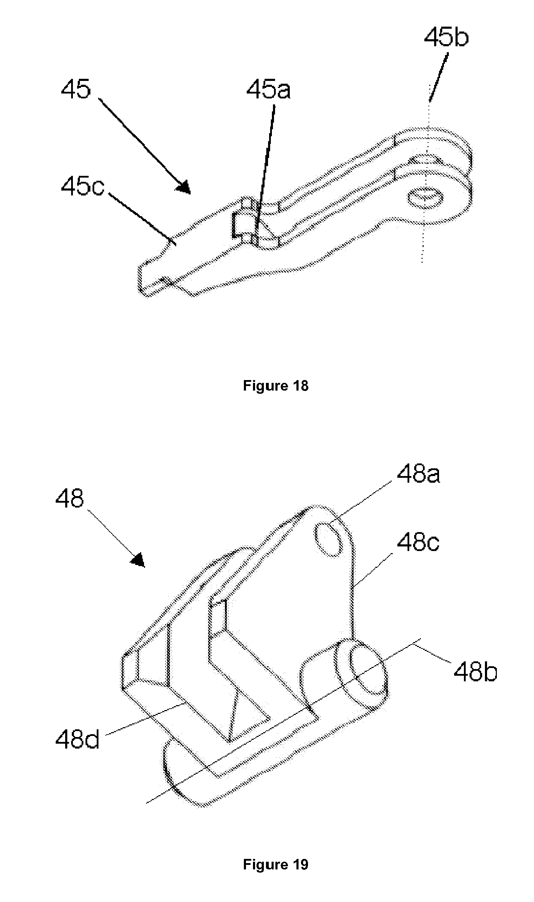

The cartridge 49 comprises a release member 48 (shown in more detail in Fig. 19) which is mounted rotationally movable around an axis 48b in the cartridge. The release member 48 is movable at least between a first (inactive) position and a second (active) position. In both positions, the release member 48 is held by protrusions 48a cooperating with pockets 49a and 49b in the cartridge body. In another embodiment, the release member comprises pockets, whereas the cartridge comprises protrusions.

The surgical stapler comprises a blocking member 45 (shown in more detail in Fig. 18) which is mounted rotationally movable around an axis 45b in the stapler housing. The blocking member 45 is movable at least between a first (blocking) position and a second (non-blocking) position. A spring 46 biases the blocking member 45 towards the first (blocking) position.

In the second (active) position, the release member body 48c is extending proximally from the axis 48b, thereby interacting with the distal portion 45c of a blocking member 45 and pushing the blocking member 45 against the force of the spring 46 towards the second (non-blocking) position.

With the blocking member 45 in the second (non-blocking) position the cutting means 43 is free to be moved distally to cut tissue clamped into the surgical stapler. Upon distal movement of the cutting means 43, the distal end portion of the cutting means contacts the release member body 48c and - during further movement - pushes the release member 48 from the second (active) position into the first (inactive) position where it is held by interaction of the protrusions 48a with pockets 49b.

Since in the first (inactive) position the release member 48 still extends upwards from its axis of rotation 48b, a slot 48d is provided in the release member body 48c in order to allow unobstructed movement of the cutting means 43 (see Fig. 19).

In Fig. 17, the cutting means 43 is shown in its final position after operation of the surgical stapler. The release member 48 has been rotated from its second (active) position into its first (inactive) position, in which the release member body 48c is extending distally from the axis 48b.

Therefore, the release member body 48c no longer interacts with the distal portion 45c of the blocking member 45 and the blocking member 45 is pushed into its first (blocking) position by the spring 46.

Distal movement of the cutting means 43 now is only possible up to a position in which a shoulder 43a of the cutting means 43 contacts a blocking portion 45a of the blocking member. In this position, the blade 44 of the cutting means is located inside the surgical stapler such that it does not cut tissue clamped into the surgical stapler.

It should be recognized that a number of variations of the above-identified embodiments will be obvious to one of ordinary skill in the art in view of the foregoing description. Accordingly, the invention is not to be limited by those specific embodiments and methods of the present invention shown and described herein. Rather, the scope of the invention is to be defined by the following claims and their equivalents.

A surgical stapler according to an embodiment of the present invention comprises a lockout mechanism which prevents usage of the surgical stapler with no or a used staple cartridge present so that the surgical stapler can not be used without staples. The cutting means of a surgical stapler according to an embodiment of the present invention can only be activated and moved to cut a patient's organ after the organ has already been closed by means of one or more rows of staples on both sides of the cutting means. Thus, a patient cannot be severely harmed, and his or her life put in danger, by leakage of fluids from a resected organ which has not been stapled before.

## Claims

1. A surgical stapler for applying at least two rows of staples, said rows of staples being housed in a cartridge having a slot extending along the cartridge between two rows of staples, said stapler comprising
a cutting means movable from an initial position, in which the distal end of said cutting means is located proximally of said rows of staples, along said slot and
a lockout mechanism for preventing movement of said cutting means,
**characterized in that**
said lockout mechanism comprises a blocking member movable between a first position, in which the blocking member blocks movement of said cutting means, and a second position, in which the blocking member permits movement of said cutting means, and
said lockout mechanism further comprises a biasing member for biasing said blocking member towards said first position.

2. The surgical stapler according to claim 1, wherein said cartridge comprises a release member for moving said blocking member towards said second position upon insertion of said cartridge into said stapler.

3. A surgical stapler for applying at least two rows of staples, said stapler comprising a cartridge housing said rows of staples and having a slot extending along the cartridge between two rows of staples, said stapler further comprising a cutting means movable along said slot and a lockout mechanism for preventing movement of said cutting means,
**characterized in that**
said lockout mechanism comprises a blocking member movable between a first position, in which the blocking member blocks movement of said cutting means, and a second position, in which the blocking member permits movement of said cutting means, and a biasing member for biasing said blocking member towards said first position and **in that** said cartridge comprises a release member for moving said blocking member towards said second position upon insertion of said cartridge into said stapler.

4. The surgical stapler according to one of claims 1 - 3, wherein said blocking member is rotationally mounted in the surgical stapler.

5. The surgical stapler according to claim 4, wherein the blocking member comprises a blocking portion and the cutting member comprises a shoulder, wherein said blocking portion and said shoulder are arranged such that the blocking portion prevents distal movement of the cutting means by contacting said shoulder of the cutting means when said blocking member is in the first position.

6. The surgical stapler according to one of claims 1 - 3, wherein said stapler further comprises a guiding element for guiding said cutting means.

7. The surgical stapler according to claim 4, wherein the blocking member is movable in a direction perpendicular to the longitudinal direction of the stapler and wherein the blocking member is guided by the guiding element.

8. The surgical stapler according to claim 6 or 7, wherein said blocking member is an essentially planar element.

9. The surgical stapler according to one of the preceding claims, wherein said biasing member is a spring engaging a peripheral end of the blocking member.

10. The surgical stapler according to one of claims 1 - 3, wherein said blocking member and/or said biasing member are wire sections.

11. The surgical stapler according to claim 10, wherein said blocking member and said biasing member are wire portions of an integral wire.

12. A staple cartridge for use with the surgical stapler according to one of the preceding claims.

13. The staple cartridge according to claim 12, wherein said release member is integrally formed with the body of said staple cartridge.

14. The staple cartridge according to claim 12, wherein said release member is a separate component.

15. The staple cartridge according to claim 12 - 14, wherein said release member is deactivatable by means of longitudinal movement of said cutting means by plastically bending said release member with respect to the body of said staple cartridge.
